# EUROPEAN PATENT APPLICATION

(11) **EP 2 769 989 A1**
(43) Date of publication of application: **27.08.2014**
(21) Application number: 13156069.0
(22) Date of filing: 21.02.2013
(51) Int. Cl.: C07K 16/28

(54) **Recombinant bispecific antibody that binds to the CD133 antigen on tumor cells and to the human CD3 T cell receptor**

(71) Applicant: Universitätsklinikum Freiburg, 79106 Freiburg (DE)
(72) Inventor: Niedermann, Gabriele, 79104 Freiburg (DE); Prasad, Shruthi, 79106 Freiburg (DE); Gaedicke, Simone, 79353 Bahlingen am Kaiserstuhl (DE); Hettich, Michael, 79359 Riegel (DE); Firat, Elke, 79104 Freiburg (DE)
(74) Representative: Keller, Günter

(57) **Abstract**

The present invention relates to recombinant bispecific antibodies that bind specifically to the CD133 surface antigen, which is expressed on tumor cells and that bind also to T cells via the CD3 receptor.

## Description

### Technical Field

The present invention relates to recombinant bispecific antibodies that bind specifically to the CD133 surface antigen, which is expressed on tumor cells and that bind also to T cells via the CD3 receptor.

In recent years tremendous progress has been made in the therapy of patients suffering from tumors. However, treatment of tumors with chemotherapy has frequently undesired side effects since also healthy cells are affected and a substantial improvement of the health cannot always be reached. Frequently, the surgical therapy by removing the primary tumor is helpful for the patient but only for a short period of time. According to the cancer stem cell (CSC) hypothesis, tumors are formed and maintained by a rare population of undifferentiated cells that are characterized by their ability to self-renew and induce tumorigenesis. Frequently, metastases of the tumors are initiated by such tumorigenic cells.

### Prior Art

In the literature it has been reported (Bidlingmaier et al., J. Mol. Med. (2008) 86(9) pp. 1025-1032) that cancer stem cells express CD133 and that a particular epitope designated AC133 is specific for cancer stem cells.

The pentaspan membrane protein CD133 has been used for CSC identification in several types of malignant tumors, such as glioblastoma, melanoma, liver cancer, osteosarcoma and colon cancer. The human CD133 gene contains at least 37 exons spanning 152 kB on chromosome 4 and several alternatively spliced forms of CD133 have been described in humans. CD133 has three extracellular domains, five transmembrane domains and three intracellular domains. Therefore, only a part of the molecule is present on the outside of the cell and antibody derivatives which are used for therapeutic and/or diagnostic purposes should be directed to the extracellular domains of CD133. Moreover, the molecule is glycosylated and the best epitopes for therapeutic and/or diagnostic purposes seem to be dependent of glycosylation which means that the antibody binding fragment binds to a glycosylation-dependent extracellular epitope of CD133.

In the prior art, antibodies directed against an epitope of CD133 are disclosed. US 2010/0209439 discloses anti-prominin-1 antibodies having antibody-dependent cellular cytotoxicity (ADCC) activity or complement-dependent cytotoxicity (CDC) activity. WO 2010/119119 discloses trifunctional bispecific antibodies against EpCAM and CD3 for the therapeutic eradication of EpCAM+ cancer stem cells some of which co-express CD133.

### The Present Invention

One object of the invention is the elimination of AC133+ tumor (stem) cells using recombinant bispecific T cell-recruiting antibodies. The preferred bispecific AC133xCD3-antibody recognizes the tumor stem cell marker AC133 and the CD3-chain of the T cell receptor on the surface of T lymphocytes. The binding of the bispecific antibody to T cells and AC133+ tumor cells induces T cell activation and specific and fast lysis of AC133+ tumor cells. AC133 (a glycosylation-dependent epitope of CD133) is a well characterized tumor stem cell marker. Within the last years, this epitope has been described as a tumor stem cell marker for a large number of tumor entities. It is assumed that many tumors, similar to normal tissues, have a hierarchical structure with tumor stem cells at the top of the hierarchy. Tumor stem cells are believed to be essential for tumor progression as well as for tumor invasion and metastasis. Generally, tumor stem cells are more resistant to conventional tumor therapies like radio- or chemotherapy compared to more differentiated tumor cells. Therefore new methods are urgently needed to specifically eradicate tumor stem cells.

The present invention provides in the broadest scope recombinant bispecific antibodies which bind to two different antigens. One arm binds to the AC133 epitope of the CD133 protein on tumor cells and the other arm binds to the CD3 receptor on T cells.

A typical antibody has a Y-shaped form which can be dissected in different parts. An antibody has two Fab fragments and one Fc fragment. In the present case, the recombinant bispecific antibodies do not have an Fc fragment.

Each Fab fragment consists of a part derived from the heavy chain and a part derived from the light chain. Each of the two parts has a constant part and a variable region. In the present invention, the constant part is of less importance. The most important aspect of the recombinant bispecific antibodies are the variable regions. The recombinant bispecific antibodies can have at least one variable region. Preferably it contains, however, two regions whereby one variable region is derived from the heavy chain (V_{H}) and the other variable region is derived from the light chain (V_{L}). Since the recombinant antibody constructs are bispecific, the variable regions bind on the one hand to AC133/CD133 and on the other hand to CD3 on the T cell receptor.

The idea of binding to the CD3 T cell receptor is that the recombinant bispecific antibody forms a link between the tumor cells or preferably the tumor stem cells and the T cells which kill the undesired tumor cells by secreting cytotoxic proteins.

Depending on the mode of application, the recombinant bispecific antibody can be administered to a patient. One of the problems which may occur is that the recombinant bispecific antibodies are very quickly excreted from the body of the patient and accumulate in excreting organs like the kidney. One option to overcome this problem is to isolate *ex vivo* T cells from the blood of the patients for example by leukapheresis and to incubate T cells with the diabody. Such incubated mixtures can thereafter be re-administered to the patient.

In particular embodiments, like tumors occurring in the brain, the recombinant bispecific antibody has to cross the blood-brain barrier. This may be possible since the recombinant bispecific antibodies disclosed herein are comparatively small. An alternative method of application is to apply the diabody into the tumor either by injection or continuously with the help of specifically adapted continuous peristaltic pumps.

In a preferred embodiment, the recombinant bispecific antibody binds to a specific epitope on CD133. This epitope is designated AC133.1. In another preferred embodiment, the recombinant bispecific antibody of the present invention does not bind to EpCAM. The abbreviation EpCAM stands for epithelial cell adhesion molecule. This is a protein that is expressed in almost all carcinoma. According to the present invention, it is, however, desired to avoid that the recombinant bispecific antibody binds to EpCAM.

The recombinant bispecific antibodies of the present invention can be used for the treatment of malignant tumors whereby the construct makes use of the own defense measures of the body, namely the cytotoxic T cells. These recombinant bispecific antibodies are particularly useful for the treatment of malignant tumors selected from the group consisting of glioblastoma, melanoma, osteosarcoma, leukemia as well as many different kinds of cancer including liver, pancreatic, and colon cancer. A particularly useful application of this bispecific antibody is the treatment of malignancies occurring in the brain, in particular glioblastoma.

In a further embodiment the recombinant bispecific antibodies of the present invention may additionally be provided with a marker which allows the detection of the construct in the body. Such a marker may be provided by radioactivity. The antibody can for example be marked with H³ (tritium) or C¹⁴. Alternatively, the construct may also be linked with a fluorescent dye or a molecule which may be detected by suitable detection methods used in medicine. The construct may comprise for example a sequence comprising an epitope which can be identified with the help of a specific (monoclonal) antibody. Such marker may be used for imaging.

In contrast to the disclosure of the international patent application WO 2010/119119, the recombinant bispecific antibodies of the present invention do not bind to EpCAM. EpCAM is a marker for tumor cells of epithelial origin (carcinoma cells). Some EpCAM+ carcinoma stem cells co-express CD133 and carcinoma stem cells double-positive for EpCAM and AC133/CD133 can thus be eliminated by T cell-recruiting antibodies targeting EpCAM. However, carcinoma stem cells which are EpCAM negative but CD133 positive can in the presence of EpCAMxCD3 antibodies (WO 2010/119119) not be killed. Moreover, nonepithelial malignancies such as glioblastoma, medulloblastoma, ependymonema, melanoma, sarcoma and the different forms of leukemia cannot be treated with EpCAMxCD3-specific antibodies since those types of tumors do not contain EpCAM-positive tumor cells.

As used herein, a "recombinant bispecific antibody" denotes a single polypeptide chain comprising two binding domains. Each binding domain comprises one variable region from an antibody heavy chain ("VH region"), wherein the VH region of the first binding domain specifically binds to the CD3 molecule, and the VH region of the second binding domain specifically binds to the extracellular domain of a membrane protein on a target cell, CD133. The two binding domains are optionally linked to one another by a short polypeptide spacer. A non-limiting example for a polypeptide spacer is Gly-Gly-Gly-Gly-Ser (G-G-G-G-S) and repeats thereof. Each binding domain may additionally comprise one variable region from an antibody light chain ("VL region"), the VH region and VL region within each of the first and second binding domains being linked to one another via a polypeptide linker. Such linker must in any case be long enough to allow the VH region and VL region of the first binding domain and the VH region and VL region of the second binding domain to pair with one another such that, together, they are able to specifically bind to the respective first and second binding domains.

In a preferred embodiment, the recombinant bispecific antibodies are diabodies which are a new class of small bivalent and bispecific antibody fragments that can be expressed in bacteria (e.g. *E.coli*)*,* yeast (e.g. *Pichia pastoris*)*,* and cells from higher eukaryotic organisms in functional form and with high yields (up to 1g/l).

Diabodies comprise a heavy (VH) chain variable domain connected to a light chain variable domain (VL) on the same polypeptide chain (VH-VL) connected by a peptide linker that is too short to allow pairing between the two domains on the same chain. This forces pairing with the complementary domains of another chain and promotes the assembly of a dimeric molecule with two functional antigen binding sites.

To construct bispecific diabodies, the variable domains derived from antibody A and antibody B are fused to create the two chains VHA-VLB, VHB-VLA. Each chain is inactive in binding to antigen, but recreates the functional antigen binding sites of antibodies A and B on pairing with the other chain. When the two chains are not linked by a spacer care has to be applied when the diabody is produced to obtain a high yield of functional products.

The term "protein" is well known in the art and describes biological compounds. Proteins comprise one or more amino acid chains (polypeptides), whereby the amino acids are bound among one another via a peptide bond. The term "polypeptide" as used herein describes a group of molecules, which consists of more than 30 amino acids. In accordance with the invention, the group of polypeptides comprises "proteins" as long as the proteins consist of a single polypeptide chain. Also in line with the definition, the term "polypeptide" describes fragments of proteins as long as these fragments consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a heteromultimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is effected e.g. by post-translational modifications like glycosylation, acetylation, phosphorylation and the like. Such modifications are well known in the art. The glycosylation of CD133 is very important.

The term "binding domain" characterizes in connection with the present invention a domain of a polypeptide which specifically binds to/interacts with a given target structure/antigen/epitope. Thus, the binding domain is an "antigen-interaction-site".

The term "antigen-interaction-site" defines, in accordance with the present invention, a motif of a polypeptide, which is able to specifically interact with a specific antigen or a specific group of antigens, e.g. the identical antigen in different species. Said binding/interaction is also understood to define a "specific recognition". The term "specifically recognizing" means in accordance with this invention that the antibody molecule is capable of specifically interacting with and/or binding to at least two, preferably at least three, more preferably at least four amino acids of an antigen, e.g. the human CD3 antigen and the other target antigen CD133.

Such binding may be exemplified by the specificity of a "lock-and-key-principle". Thus, specific motifs in the amino acid sequence of the binding domain and the antigen bind to each other as a result of their primary, secondary or tertiary structure as well as as the result of secondary modifications of said structure. The specific interaction of the antigen-interaction-site with its specific antigen may result as well in a simple binding of said site to the antigen. Moreover, the specific interaction of the binding domain/antigen-interaction-site with its specific antigen may alternatively result in the initiation of a signal, e.g., due to the induction of a change of the conformation of the antigen, an oligomerization of the antigen, etc.

The term "antibody" relates to derivatives or functional fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also comprises immunoglobulins (Ig's) of different classes (i.e. IgA, IgG, IgM, IgD and IgE) and subclasses (such as IgG1, IgG2 etc.).

The definition of the term "antibody" also includes embodiments such as chimeric, single chain and humanized antibodies, as well as antibody fragments, like, inter alia, Fab fragments. Antibody fragments or derivatives further comprise F(ab')₂, Fv, scFv fragments or single domain antibodies, single variable domain antibodies or immunoglobulin single variable domain comprising merely one variable domain, which might be VH or VL, that specifically bind to an antigen or epitope independently of other V regions or domains. Such immunoglobulin single variable domain encompasses not only an isolated antibody single variable domain polypeptide, but also larger polypeptides that comprise one or more monomers of an antibody single variable domain polypeptide sequence.

The term "specific interaction" as used in accordance with the present invention means that the binding domain does not or does not significantly cross-react with polypeptides which have similar structure as those bound by the binding domain, and which might be expressed by the same cells as the polypeptide of interest. Cross-reactivity of a panel of binding domains under investigation may be tested, for example, by assessing binding of said panel of binding domains under conventional conditions (see, e.g., Harlow and Lane, Antibodies: A Laboratory Manual, Cold Spring Harbor Laboratory Press, 1988 and Using Antibodies: A Laboratory Manual, Cold Spring Harbor Laboratory Press, 1999). Examples for the specific interaction of a binding domain with a specific antigen comprise the specificity of a ligand for its receptor. Said definition particularly comprises the interaction of ligands, which induce a signal upon binding to its specific receptor. Examples for said interaction, which is also particularly comprised by said definition, is the interaction of an antigenic determinant (epitope) with the binding domain (antigenic binding site) of an antibody.

According to a preferred embodiment of the method of the invention one binding domain binds to CD3 epsilon (CD3_{ε}) of human T cells.

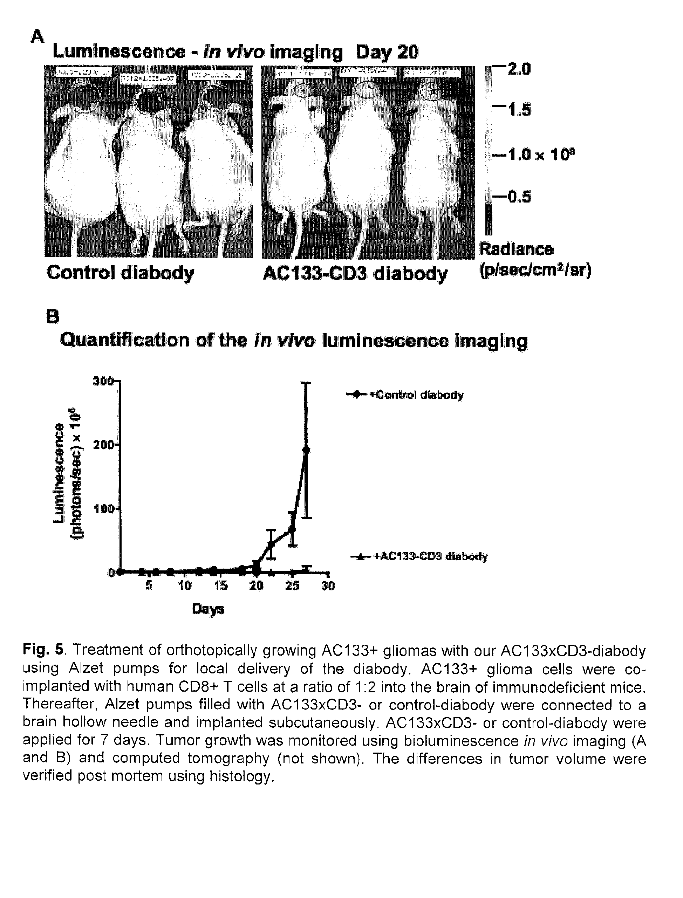

Considerable advantages arise for the treatment of brain tumors with AC133+ tumor stem cells. These tumors include *Glioblastoma multiforme* (WHO grade IV) and other lower grade gliomas, medulloblastoma, ependymoma, pineoblastoma and teratoid/rhabdoid tumors. These brain tumors belong to the most malignant tumor entities and have a poor prognosis. For instance, *Glioblastoma multiforme,* the most common brain tumor in adults, still remains incurable and standard treatment with surgical resection plus radiochemotherapy gives a median survival of only one year. The other mentioned tumor entities have a poor prognosis as well. New therapeutic modalities, especially so called "targeted therapies" to which in particular the preferred AC133xCD3-diabody belongs, are therefore urgently needed. The experimental studies in orthotopic brain tumor models using human AC133+ glioma cells in immunodeficient mice show that local treatment with AC133xCD3-diabody using pressure-backed infusion with Alzet osmotic pumps has an excellent antitumoral effect (see Fig. 5). In a similar fashion, patients with AC133+ brain tumors could be treated locally using "Convection Enhanced Delivery" (CED). Although higher concentrations of the diabody can be achieved in the brain tumor by local treatment, systemic application of the diabody could also prove effective. However, the efficiency of systemic therapy of brain tumors is usually restricted by the blood-brain barrier.

In summary, the preferred AC133xCD3-diabody is a T cell-recruiting antibody, which specifically recognizes AC133+ tumor stem cells and induces their T cell-dependent destruction. Moreover the AC133xCD3-diabody could be of great importance for the local treatment of aggressive and deadly brain tumors such as glioblastoma which contain AC133+ tumor stem cells. The experiments with immunodeficient mice show that local therapy with the AC133xCD3-diabody is highly efficient, and considering the fact that postnatally AC133 is not expressed in the brain, it can be expected that local application of the diabody will not cause significant side effects.

Antibodies useful in the methods of the present invention can be generated using various phage display methods known in the art. In phage display methods, functional antibody domains are displayed on the surface of phage particles which carry the nucleic acid sequences encoding them. In a particular embodiment, such phage can be utilized to display antigen binding domains expressed from a repertoire or combinatorial antibody library (e.g., human or murine). In particular, DNA sequences encoding V_{H} and V_{L} domains are amplified from animal cDNA libraries (e.g., human or murine cDNA libraries of lymphoid tissues). The DNA encoding the V*_{H}* and V*_{L}* domains are recombined together with an scFv linker by PCR and cloned into a phagemid vector. The vector is electroporated in *E. coli* and the *E. coli* is infected with helper phage. Phage used in these methods are typically filamentous phage including fd and M13 binding domains expressed from phage with Fab, Fv or disulfide stabilized Fv antibody domains recombinantly fused to either the phage gene III or gene VIII protein. Phage expressing an antigen binding domain that binds to or-portions thereof can be selected or identified with antigen e.g., using labeled antigen or antigen bound or captured to a solid surface or bead.

Alternatively it is also possible to produce the recombinant bispecific antibody constructs of the present invention by hybridoma fusion.

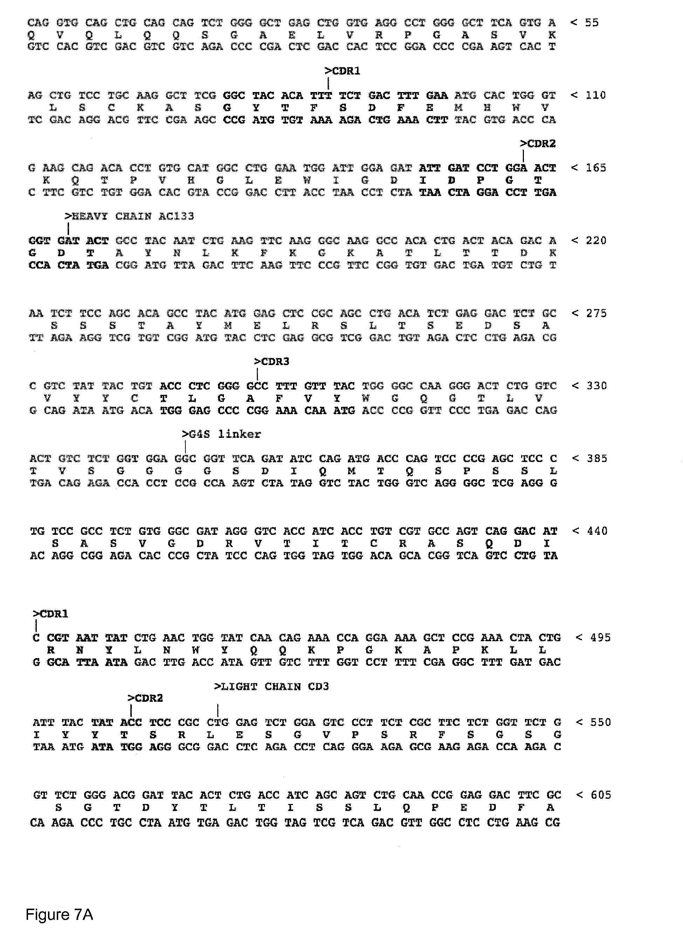

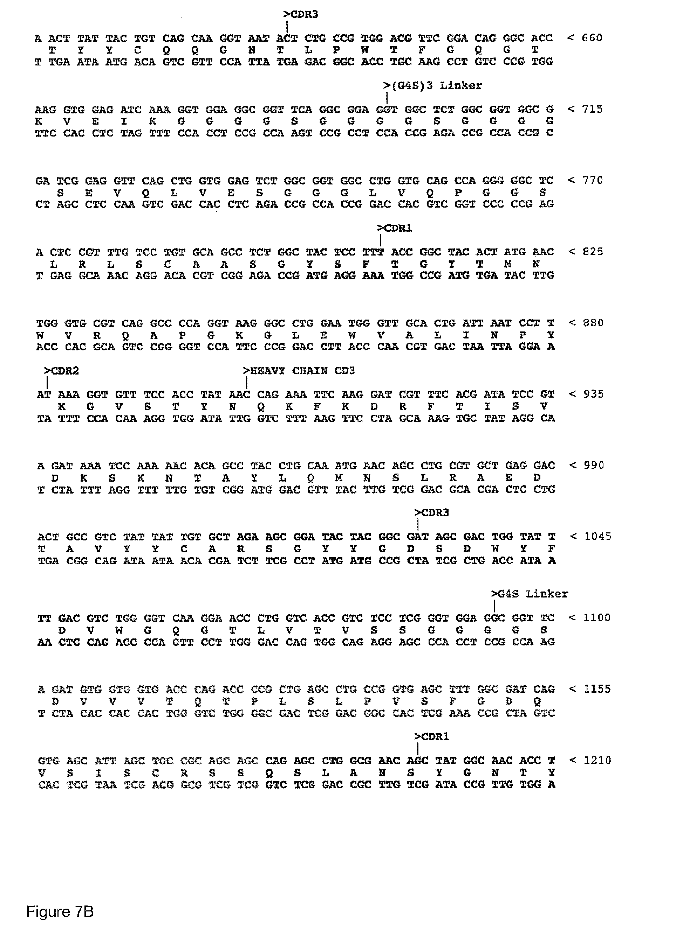

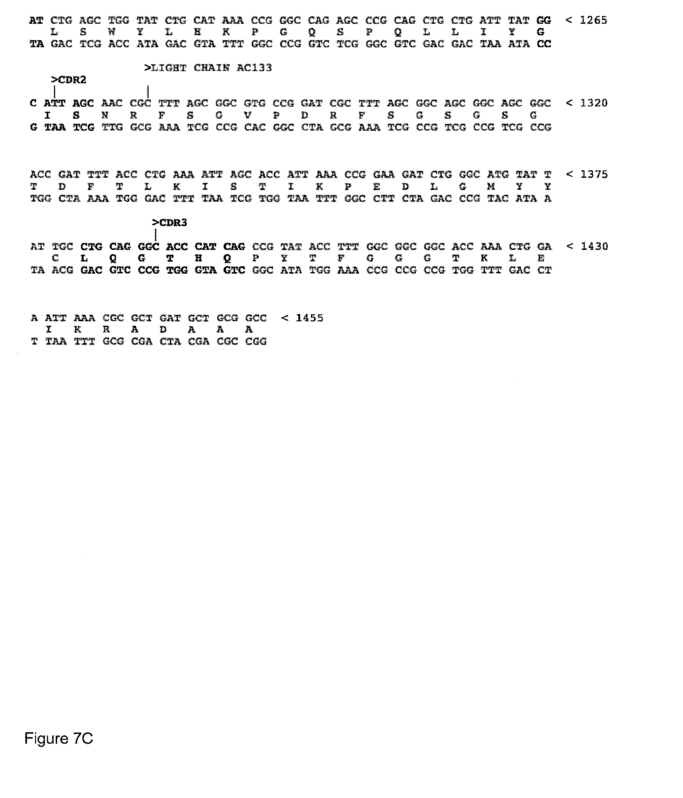

The present invention is further illustrated by the figures. In particular, the figures disclose also preferred embodiments of the invention:

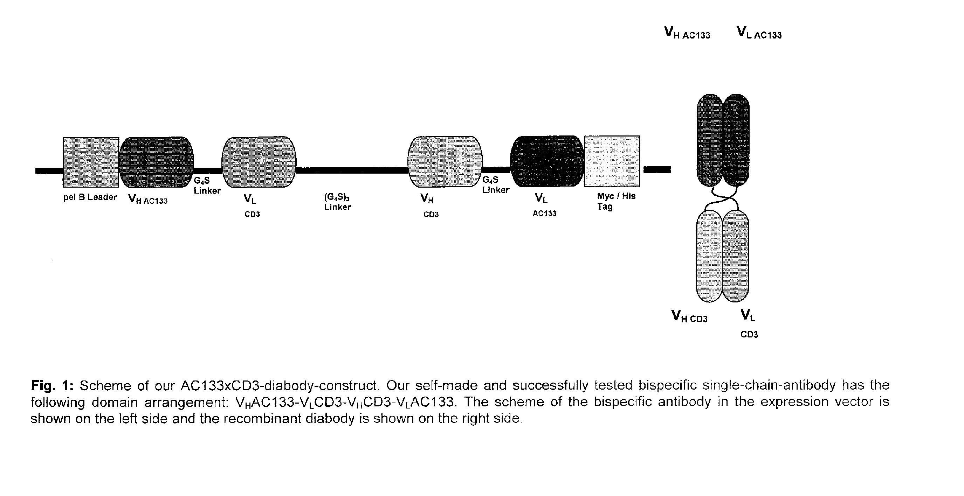

**Fig. 1****:** Scheme of a preferred AC133xCD3-diabody-construct. The successfully tested bispecific single-chain-antibody has the following domain arrangement: \/_{H}AC133-V_{L}CD3-V_{H}CD3-V_{L}AC133. The scheme of the bispecific antibody in the expression vector is shown on the left side and the recombinant diabody is shown on the right side.
   Constructs with other domain arrangements should also work.

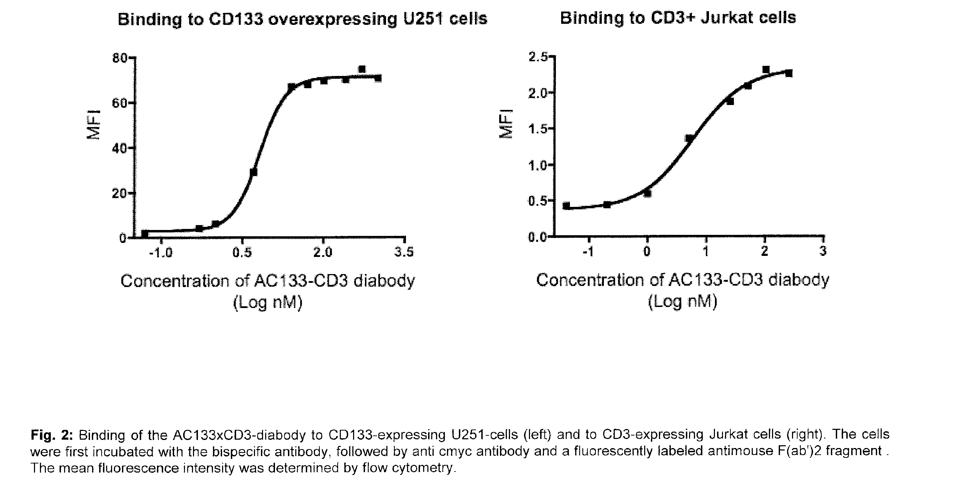

**Fig. 2****:** Binding of the AC133xCD3-diabody to CD133-overexpressing U251 glioma cells (left) and to CD3-expressing Jurkat cells (right). The cells were first incubated with the bispecific antibody, followed by anti-Myc antibody and a fluorescently labeled anti-mouse F(ab')₂ fragment. The fluorescence signals were determined by flow cytometry.

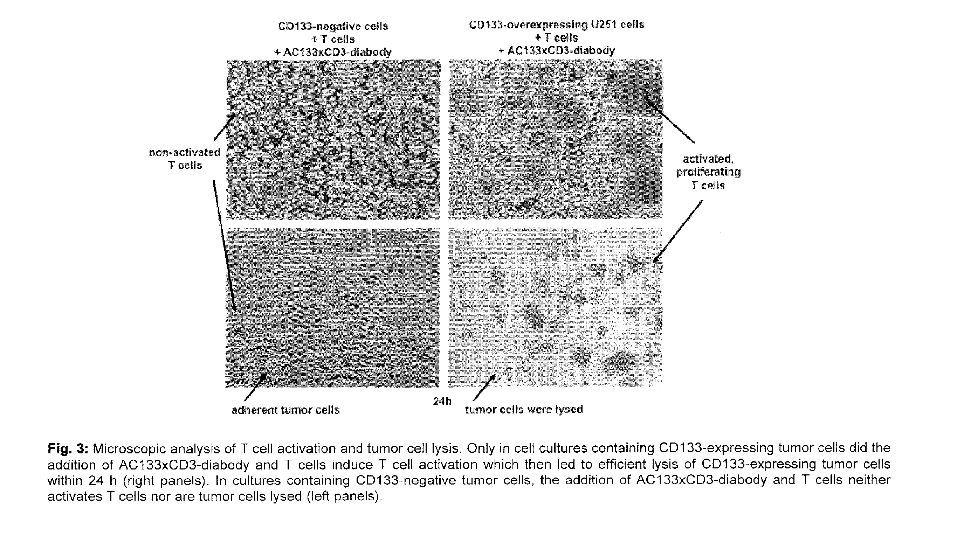

**Fig. 3****:** Microscopic analysis of T cell activation and tumor cell lysis. Only in cell cultures containing CD133-expressing tumor cells did the addition of AC133xCD3-diabody and T cells induce T cell activation which then led to efficient lysis of CD133-expressing tumor cells within 24 h (right panels). In cultures containing CD133-negative tumor cells, the addition of AC133xCD3-diabody and T cells neither activates T cells nor are tumor cells lysed (left panels). This figure shows that the bispecific antibody works *in vitro.* Similar results were obtained with primary, patient-derived AC133+ tumor stem cells (not shown).

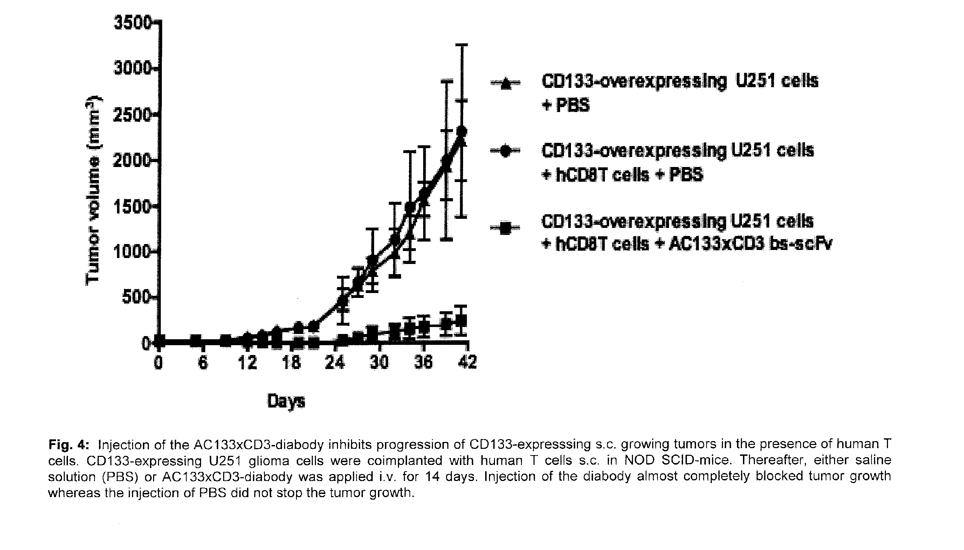

**Fig. 4****:** Injection of the AC133xCD3-diabody inhibits progression of CD133-expresssing s.c. growing tumors in the presence of human T cells. CD133-overexpressing U251 glioma cells were coimplanted with human T cells s.c. in NOD SCID-mice. Thereafter, either saline solution (PBS) or AC133xCD3-diabody was applied i.v. for 14 days. Injection of the diabody almost completely blocked tumor growth whereas the injection of PBS did not stop the tumor growth. This figure shows that the bispecific antibody works *in vivo* in an animal model. Similar results were obtained with primary, patient-derived AC133+ tumor stem cells (not shown).
**Fig. 5****.** Treatment of orthotopically growing AC133+ gliomas with our AC133xCD3-diabody using Alzet pumps for local delivery of the diabody. AC133+ glioma cells were coimplanted with human CD8+ T cells at a ratio of 1:2 into the brain of immunodeficient mice.
Thereafter, Alzet pumps filled with AC133xCD3- or control-diabody were connected to a brain hollow needle and implanted subcutaneously. AC133xCD3- or control-diabody were applied for 7 days. Tumor growth was monitored using bioluminescence *in vivo* imaging (A and B) and computed tomography (not shown). The differences in tumor volume were verified post mortem using histology. This figure is further evidence that the invention works *in vivo.*

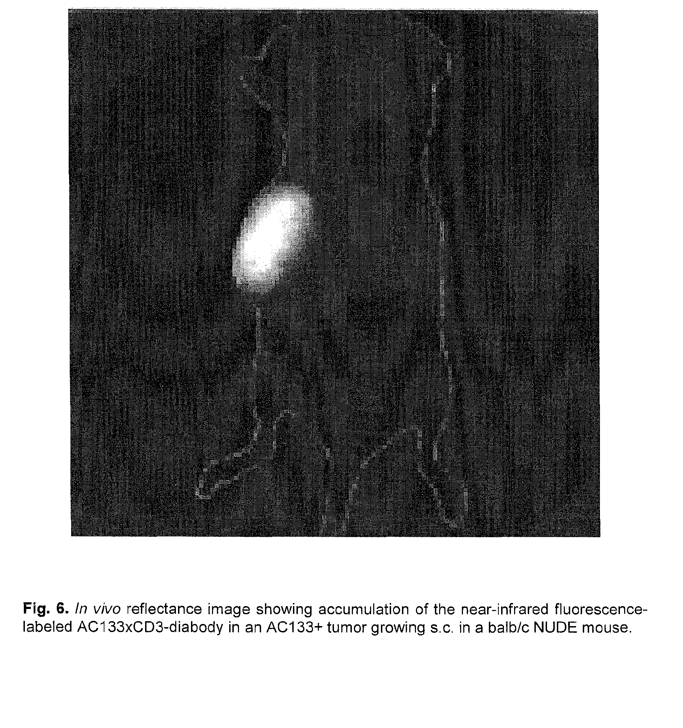

**Fig. 6****.** *In vivo* reflectance image showing accumulation of the near-infrared fluorescence-labeled AC133xCD3-diabody in an AC133+ tumor growing s.c. in a balb/c NUDE mouse. This figure demonstrates a preferred embodiment wherein the antibody is tagged with a marker suitable for imaging.
   The V_{H} und V_{L} sequences of the AC133 antibody were selected by phage display using the cDNA of the AC133-hybridoma (ATCC HB-12346). V_{H} and V_{L} sequences of CD3 were obtained from the published sequence of the humanized UCHT1 antibody. The bispecific construct was gene-synthesised (Genscript, USA) in the following configuration: V_{H}AC133-G₄S Linker-V_{L}CD3- (G₄S)₃ Linker-V_{H}CD3- G₄S Linker -V_{L}AC133
**Fig. 7****.** Figures 7A, 7B and 7C show the sequence of the preferred embodiment. The forward strand sequence (SEQ ID NO:1) is shown as well as the complementary strand (reverse strand) (SEQ ID NO:2) and the amino acid sequence (SEQ ID NO:3). Moreover, the CDRs responsible for the binding to the antigens are shown. In Figure 7A, CDR1 (SEQ ID NO:4), CDR2 (SEQ ID NO:5) and CDR3 (SEQ ID NO:6) of the heavy chain of the AC133 part are shown in the upper part of Figure 7A. In the lower part of Figure 7A, CDR1 (SEQ ID NO:7) and CDR2 (SEQ ID NO:8) of the light chain binding to CD3 are shown. In Figure 7B upper part the CDR3 relating to light chain CD3 is shown (SEQ ID NO:9). In the middle of Figure 7B the CDRs of the heavy chain binding to CD3 can be found, in particular CDR1 (SEQ ID NO:10), CDR2 (SEQ ID NO:11) and CDR3 (SEQ ID NO:12). In the lower part of Figure 7B and in Figure 7C CDR1 (SEQ ID NO:13), CDR2 (SEQ ID NO:14) and CDR3 (SEQ ID NO:15) of the light chain binding to AC133 can be found.

In a preferred embodiment a recombinant bispecific antibody according to the present invention comprises the above-mentioned CDRs (SEQ ID NO:4 to SEQ ID NO:15) since those areas are essential for an effective binding of the recombinant bispecific antibody. The SEQ ID NO:4 to 15 show the nucleic acid sequences. The corresponding amino acid sequences can be deduced from Fig. 7. The framework region may be slightly amended without negatively influencing the binding properties of the construct. Such modifications may be single substitutions of amino acids located in the framework region and/or the linker areas. Such modifications may be performed in order to improve the biological acceptance of the construct. Amino acid replacements may serve the purpose to make the framework regions more similar to sequences naturally occurring in humans in order to avoid undesired immunological reactions against the artificial construct, in particular the framework regions.

The present invention provides recombinant bispecific antibodies which have the unique binding properties as shown in the present application. It is essential that the recombinant bispecific antibodies comprise the CDRs as shown in Figure 7. The coding regions are shown in Figure 7 and the nucleic acid sequence is provided as SEQ ID NO:4 to SEQ ID NO:15. Since the CDRs define the binding characteristics of the recombinant antibody construct it is preferred that a recombinant bispecific antibody according to the present invention comprises at least nine, more preferred at least 11 sequences encoded by any of SEQ ID NO:4 to SEQ ID NO:15. In a more preferred embodiment the recombinant bispecific antibody comprises as CDRs the amino acid sequences encoded by each of the SEQ ID NO:4 to SEQ ID NO: 15.

In a particularly preferred embodiment the recombinant bispecific antibody comprises the amino acid sequence as provided as SEQ ID NO:3.

The person skilled in the art is aware of the fact that minor changes of the amino acid sequence are possible as long as the changes are in the framework region. It is common general knowledge to humanize the framework region. In the process of humanization of a recombinant construct, amino acids which are typical for the mouse framework are replaced by similar amino acids which resemble as closest possible human framework structures.

The invention is further illustrated by the following examples:

### Example 1

The AC133-CD3 bispecific antibody construct was cloned into the pOPE 101 expression vector (Progen) and transformed into E.coli XL1 Blue competent cells (Stratagene). The positive recombinant clones were selected by ampicillin selection and the clones were grown overnight at 37°C in 2YT medium containing 100 µg/mL ampicillin and 100 mM glucose. The overnight culture was diluted 1: 20 and grown at 37°C till the OD₆₀₀ reached 0.8. Afterwards, the culture was harvested and resuspended in 2YT medium containing 50 µg/mL ampicillin, 0.4 M sucrose and 1 mM IPTG. The culture was grown at room temperature for 18-20 hours, harvested and resuspended in 5% of initial volume of ice-cold 50 mM Tris HCl (20% sucrose, 1 mM EDTA, pH 8.0) and incubated for 1 hour on ice. This was followed by centrifugation at 20,000g at 4°C for 30 minutes. The periplasmic extract was dialyzed overnight against 50 mM Tris HCl, 1 M NaCl pH 7.0. The purification was performed by Ni²⁺ affinity chromatography (Qiagen) and the purified protein was dialyzed against PBS.

The diabody can be prepared with high purity and a high yield (not shown).

### Example 2

The binding specificity of the AC133xCD3-bispecific antibody was demonstrated by flow cytometry using U251 glioma cells (ATCC) which were lentivirally transduced to overexpress CD133, cultured in DMEM (Invitrogen) with 10% FCS (PAA) supplemented with 1% penicillin/streptomycin (PAA), 1x MEM non-essential amino acids (Invitrogen) and 2 mM L-glutamate (PAA) and the CD3+ acute T cell leukemia cell line Jurkat, cultured in RPMI 1640 (Invitrogen) with 10% FCS (PAA) supplemented with 1% penicillin/streptomycin (PAA), 1x MEM non-essential amino acids (Invitrogen). The cells were harvested, counted and resuspended in FACS buffer (PBS containing 0.5% BSA and 2mM EDTA). 2.5x10⁵ target cells were incubated with different concentrations of the bispecific antibody for 45 minutes on ice. This was followed by two wash steps with FACS buffer and incubation with 1 µg/mL of anti-c Myc Mouse mAb (Calbiochem) for 45 minutes on ice. After the incubation, the cells were washed twice with FACS buffer and incubated with 1.5 µg of anti-mouse PE-conjugated F(ab')₂ fragment (Dianova) for 30 min. After two washes with FACS buffer, the cells were checked and analysed on a Cytomix FC-500 flow cytometer (Beckman Coulter, Krefeld, Germany). The mean fluorescence intensity (MFI) was plotted using GraphPad Prism (GraphPad Software) and dissociation constant (Kd) values determined.

Binding of the purified antibody was highly specific to both AC133-positive and CD3-positive cells. Results are shown in Figure 2. The Kd value of the bispecific antibody was determined by FACS based mean fluorescence intensity analysis and was found to be 6.4 nM.

### Example 3

The efficiency of the bispecific antibody to redirect T cells thereby killing antigen-expressing cells was determined using a FACS-based cytotoxicity assay. The target cells were labeled with PKH26 (Sigma) according to the manufacturer's instructions. 0.5x10⁵ target cells were plated in a 48 well plate and allowed to adhere. Human CD8+ T cells were added at a effector to target cell ratio of 10 : 1. Different concentrations of the bispecific antibody were then added to the wells. The cells were incubated for 24 h at 37°C in a 5% CO₂ incubator. The cells were then collected. After adding 7-AAD (BD Pharmingen) at a final concentration of 0.25 µg/mL, the cells were analysed on a Cytomix FC-500 flow cytometer (Beckman Coulter, Krefeld, Germany).

AC133+ tumor cells are highly specifically and highly efficiently lysed by T cells in the presence of the AC133xCD3-diabody. In contrast, AC133-negative tumor cells are not lysed. The results are shown in Figure 3.

### Example 4

The AC133xCD3-diabody shows strong antitumor activity against AC133-positive tumor cells in *in vivo* experiments using immunodeficient mice. Tumor growth could only be suppressed when human T cells were co-injected with the AC133xCD3-diabody. Injection of PBS only or co-injection of PBS and T cells could not suppress the tumor growth. The results are shown in Figure 4. These experiments were first performed with subcutaneously (s.c.) growing AC133+ tumor cells, which were treated intravenously (i.v.) with the AC133xCD3-diabody or PBS. In more detail, 6-8 week old NOD-SCID mice (Charles River laboratories) were injected s.c. with 5x10⁶ CD133-overexpressing U251 cells. In the test group, 1x10⁶ human CD8+ T cells were co-injected s.c. and the mice received daily i.v. injections of 10 µg of the preferred AC133xCD3-bispecific antibody for 14 days. In the control groups, the mice either received a co-injection of 1x10⁶ human CD8 T cells and daily i.v. injections of PBS for 14 days or, in another control group, CD8+ T cells were not co-injected and the tumor-bearing mice received only PBS i.v. for 14 days.

### Example 5

The AC133xCD3-diabody proved also very efficient in orthotopic AC133+ brain tumor models and local application of the AC133xCD3-diabody. For this purpose, tumor cells were transduced with firefly luciferase in order to be able to non-invasively monitor the growth of the brain tumors. In this model, 1x10⁵ AC133+ glioma cells were implanted together with 2x10⁵ human CD8+ T cells into the brain of 6-8 week old NMRI nude mice (Harlan). Alzet osmotic pumps (model 1007D) were used to deliver the bispecific antibody intracranially. The pumps were filled either with the specific diabody (AC133xCD3) or with an unspecific diabody at a concentration of 250 µg/ml. The pumps deliver 0.5 µl of the diabody per hour for a period of 7 days. The mice were anesthetized by i.p. injection of a ketamine-medetomidine combination [50-75 mg/kg of Ketamine (Inresa Arzneimittel GmbH) and 1 mg/kg of Dorbene(Pfizer GmbH)]. The head of the mouse was disinfected with Kodan Forte (Schulke) and a 1 cm skin incision was made along the centre of the skull with a sterile disposable scalpel. Thereafter, a 3 mm hole was drilled into the skull with a sterican needle (0,40x20 mm 27Gx3/4" Gr. 20) 3 mm anterior and 3 mm to the right of the bregma. The cells were injected into this hole at a depth of 3 mm with a Hamilton syringe. The syringe was held in position for 5 minutes for injection. After the injection, the surface was cleaned with a sterile cotton swab. The filled pumps were connected to the brain infusion cannula (Brain infusion kit 3, 1-3mm from Alzet) according to the manufacturer's instructions. The pumps were placed subcutaneously. Thereafter, the brain infusion cannula was placed into the injection site at a depth of 3 mm deep and fixed to the skull using cyanoacrylate adhesive (Alzet). The incision was sutured and sprayed with Opsite (Smith and Nephew Medicals). To reverse the effects of the narcotics, the mice were injected i.p. with Atipamezole hydrochloride (Alzane, 1.0 mg/kg, Pfizer). Finally, the mice were injected s.c. with 1 mg/kg of the non-steroidal anti-inflammatory drug Metacam (Boehringer Ingelheim). Thereafter, the tumor growth was monitored non-invasively using in vivo bioluminescence imaging on an IVIS spectrum imaging system (Perkin Elmer) three times per week.

As shown in Fig. 5, the local infusion of the AC133xCD3-bispecific diabody suppressed the growth of AC133+ tumors almost completely. In contrast, unimpeded tumor growth was observed in animals receiving the control diabody. These great differences in tumor growth were confirmed by contrast agent-enhanced micro-computed tomography as well as by post mortem histology (not shown).

### Example 6

Using a fluorescence-labeled AC133xCD3-diabody and non-invasive fluorescence *in vivo* imaging, we could show that the AC133xCD3-diabody accumulates in AC133+ tumors. Results are shown in Figure 6. 10 µg of AC133xCD3 diabody labeled with SAIVI Alexaflour 750 dye (Invitrogen) were injected i.v. into AC133+ tumor-bearing Nude mice. Imaging was done 24 hours after the injection using the near-infrared fluorescence molecular tomography system FMT1500 from PerkinElmer.

Therefore, the AC133-specific antibody fragments are generally suitable for non-invasive *in vivo* imaging of AC133+ tumors including intraoperative or endoscopic tumor-imaging. In further experiments, it was shown that recombinant AC133-single-chain antibodies, AC133xAC133-diabodies or other antibody-fragments based on the AC133-single-chain antibody are suitable for *in vivo* imaging of AC133+ tumors. In addition to fluorescence-based *in vivo* imaging (including intraoperative or endoscopic tumor imaging), such antibody fragments can also be used for positron emission tomography, SPECT and MR imaging. In addition to the imaging of AC133+ tumor (stem) cells, the engraftment of normal autologous AC133+ stem and progenitor cells used for cell therapy for the purpose of tissue and organ regeneration (i.e. for the treatment of myocardial infarction already applied in clinical studies), could be followed up by using different imaging techniques.

## Claims

1. Recombinant bispecific antibody comprising at least one fragment having domains which bind to CD133 on tumor cells and at least one fragment having domains which bind to the human CD3 T cell receptor.

2. Recombinant bispecific antibody according to claim 1, **characterized in that** it binds to a glycosylation-dependent epitope of CD133.

3. Recombinant bispecific antibody according to claim 1 or 2, **characterized in that** it binds to CD133 expressed on tumor stem cells.

4. Recombinant bispecific antibody according to claim 3, wherein the fragment which binds on CD133 binds to the AC133.1 epitope of CD133.

5. Recombinant bispecific antibody according to any of the preceding claims, **characterized in that** it comprises a V_{H} and a V_{L} fragment which bind to CD133 and a V_{H} and a V_{L} fragment which binds to CD3.

6. Recombinant bispecific antibody according to any of the preceding claims, **characterized in that** it is a bispecific single chain antibody.

7. Recombinant bispecific antibody according to any of the preceding claims, **characterized in that** it does not bind to EpCAM.

8. Recombinant bispecific antibody according to any of the preceding claims, **characterized in that** it comprises amino acid sequences encoded by at least nine sequences selected from the group consisting of SEQ ID NO:4 to 15.

9. Recombinant bispecific antibody according to any of the preceding claims, **characterized in that** it comprises the amino acid sequence having SEQ ID NO:3.

10. Recombinant bispecific antibody according to any of the preceding claims for use in the treatment of malignant tumors.

11. Recombinant bispecific antibody according to claim 10 for use in the treatment of malignant tumors selected from the group consisting of glioblastoma, melanoma, osteosarcoma, leukemia and various kinds of carcinomas such as liver, pancreatic and colon cancer.

12. Recombinant bispecific antibody according to claim 11 for use in the treatment of glioblastoma.

13. AC133-specific antibody fragments according to any of claims 1 to 9 for use in imaging of AC133+ tumors and normal AC133+ stem or progenitor cells.


SEQUENCE LISTING

<110>  Universitätsklinikum Freiburg

<120>  Recombinant bispecific antibody that binds to the CD133 antigen
       on tumor cells and to the CD3 T cell receptor

<130>  PEP02301UNI

<160>  15

<170>  PatentIn version 3.5

<210>  1
<211>  1455
<212>  DNA
<213>  artificial construct

<220>
<223>  forward strand

<400>  1
caggtgcagc tgcagcagtc tggggctgag ctggtgaggc ctggggcttc agtgaagctg      60

tcctgcaagg cttcgggcta cacattttct gactttgaaa tgcactgggt gaagcagaca     120

cctgtgcatg gcctggaatg gattggagat attgatcctg gaactggtga tactgcctac     180

aatctgaagt tcaagggcaa ggccacactg actacagaca aatcttccag cacagcctac     240

atggagctcc gcagcctgac atctgaggac tctgccgtct attactgtac cctcggggcc     300

tttgtttact ggggccaagg gactctggtc actgtctctg gtggaggcgg ttcagatatc     360

cagatgaccc agtccccgag ctccctgtcc gcctctgtgg gcgatagggt caccatcacc     420

tgtcgtgcca gtcaggacat ccgtaattat ctgaactggt atcaacagaa accaggaaaa     480

gctccgaaac tactgattta ctatacctcc cgcctggagt ctggagtccc ttctcgcttc     540

tctggttctg gttctgggac ggattacact ctgaccatca gcagtctgca accggaggac     600

ttcgcaactt attactgtca gcaaggtaat actctgccgt ggacgttcgg acagggcacc     660

aaggtggaga tcaaaggtgg aggcggttca ggcggaggtg gctctggcgg tggcggatcg     720

gaggttcagc tggtggagtc tggcggtggc ctggtgcagc cagggggctc actccgtttg     780

tcctgtgcag cctctggcta ctcctttacc ggctacacta tgaactgggt gcgtcaggcc     840

ccaggtaagg gcctggaatg ggttgcactg attaatcctt ataaaggtgt ttccacctat     900

aaccagaaat tcaaggatcg tttcacgata tccgtagata aatccaaaaa cacagcctac     960

ctgcaaatga acagcctgcg tgctgaggac actgccgtct attattgtgc tagaagcgga    1020

tactacggcg atagcgactg gtattttgac gtctggggtc aaggaaccct ggtcaccgtc    1080

tcctcgggtg gaggcggttc agatgtggtg gtgacccaga ccccgctgag cctgccggtg    1140

agctttggcg atcaggtgag cattagctgc cgcagcagcc agagcctggc gaacagctat    1200

ggcaacacct atctgagctg gtatctgcat aaaccgggcc agagcccgca gctgctgatt    1260

tatggcatta gcaaccgctt tagcggcgtg ccggatcgct ttagcggcag cggcagcggc 1320

accgatttta ccctgaaaat tagcaccatt aaaccggaag atctgggcat gtattattgc 1380

ctgcagggca cccatcagcc gtataccttt ggcggcggca ccaaactgga aattaaacgc 1440

gctgatgctg cggcc 1455

<210> 2
<211> 1455
<212> DNA
<213> artificial construct

<220>
<223> reverse strand

<400> 2
gtccacgtcg acgtcgtcag accccgactc gaccactccg gaccccgaag tcacttcgac 60

aggacgttcc gaagcccgat gtgtaaaaga ctgaaacttt acgtgaccca cttcgtctgt 120

ggacacgtac cggaccttac ctaacctcta taactaggac cttgaccact atgacggatg 180

ttagacttca agttcccgtt ccggtgtgac tgatgtctgt ttagaaggtc gtgtcggatg 240

tacctcgagg cgtcggactg tagactcctg agacggcaga taatgacatg ggagccccgg 300

aaacaaatga ccccggttcc ctgagaccag tgacagagac cacctccgcc aagtctatag 360

gtctactggg tcaggggctc gagggacagg cggagacacc cgctatccca gtggtagtgg 420

acagcacggt cagtcctgta ggcattaata gacttgacca tagttgtctt tggtcctttt 480

cgaggctttg atgactaaat gatatggagg gcggacctca gacctcaggg aagagcgaag 540

agaccaagac caagaccctg cctaatgtga gactggtagt cgtcagacgt tggcctcctg 600

aagcgttgaa taatgacagt cgttccatta tgagacggca cctgcaagcc tgtcccgtgg 660

ttccacctct agtttccacc tccgccaagt ccgcctccac cgagaccgcc accgcctagc 720

ctccaagtcg accacctcag accgccaccg gaccacgtcg gtcccccgag tgaggcaaac 780

aggacacgtc ggagaccgat gaggaaatgg ccgatgtgat acttgaccca cgcagtccgg 840

ggtccattcc cggaccttac ccaacgtgac taattaggaa tatttccaca aaggtggata 900

ttggtcttta agttcctagc aaagtgctat aggcatctat ttaggttttt gtgtcggatg 960

gacgtttact tgtcggacgc acgactcctg tgacggcaga taataacacg atcttcgcct 1020

atgatgccgc tatcgctgac cataaaactg cagaccccag ttccttggga ccagtggcag 1080

aggagcccac ctccgccaag tctacaccac cactgggtct ggggcgactc ggacggccac 1140

tcgaaaccgc tagtccactc gtaatcgacg gcgtcgtcgg tctcggaccg cttgtcgata 1200

ccgttgtgga tagactcgac catagacgta tttggcccgg tctcgggcgt cgacgactaa 1260

ataccgtaat cgttggcgaa atcgccgcac ggcctagcga aatcgccgtc gccgtcgccg 1320

tggctaaaat gggactttta atcgtggtaa tttggccttc tagacccgta cataataacg 1380

gacgtcccgt gggtagtcgg catatggaaa ccgccgccgt ggtttgacct ttaatttgcg 1440

cgactacgac gccgg 1455

<210> 3
<211> 485
<212> PRT
<213> artificial construct

<220>
<223> diabody

<400> 3

Gln Val Gln Leu Gln Gln Ser Gly Ala Glu Leu Val Arg Pro Gly Ala
1               5                   10                  15

Ser Val Lys Leu Ser Cys Lys Ala Ser Gly Tyr Thr Phe Ser Asp Phe
            20                  25                  30

Glu Met His Trp Val Lys Gln Thr Pro Val His Gly Leu Glu Trp Ile
        35                  40                  45

Gly Asp Ile Asp Pro Gly Thr Gly Asp Thr Ala Tyr Asn Leu Lys Phe
    50                  55                  60

Lys Gly Lys Ala Thr Leu Thr Thr Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Thr Leu Gly Ala Phe Val Tyr Trp Gly Gln Gly Thr Leu Val Thr Val
            100                 105                 110

Ser Gly Gly Gly Gly Ser Asp Ile Gln Met Thr Gln Ser Pro Ser Ser
        115                 120                 125

Leu Ser Ala Ser Val Gly Asp Arg Val Thr Ile Thr Cys Arg Ala Ser
    130                 135                 140

Gln Asp Ile Arg Asn Tyr Leu Asn Trp Tyr Gln Gln Lys Pro Gly Lys
145                 150                 155                 160

Ala Pro Lys Leu Leu Ile Tyr Tyr Thr Ser Arg Leu Glu Ser Gly Val
                165                 170                 175

Pro Ser Arg Phe Ser Gly Ser Gly Ser Gly Thr Asp Tyr Thr Leu Thr
            180                 185                 190

Ile Ser Ser Leu Gln Pro Glu Asp Phe Ala Thr Tyr Tyr Cys Gln Gln
        195                 200                 205

Gly Asn Thr Leu Pro Trp Thr Phe Gly Gln Gly Thr Lys Val Glu Ile
    210                 215                 220

Lys Gly Gly Gly Gly Ser Gly Gly Gly Gly Ser Gly Gly Gly Gly Ser
225                 230                 235                 240

Glu Val Gln Leu Val Glu Ser Gly Gly Gly Leu Val Gln Pro Gly Gly
                245                 250                 255

Ser Leu Arg Leu Ser Cys Ala Ala Ser Gly Tyr Ser Phe Thr Gly Tyr
            260                 265                 270

Thr Met Asn Trp Val Arg Gln Ala Pro Gly Lys Gly Leu Glu Trp Val
        275                 280                 285

Ala Leu Ile Asn Pro Tyr Lys Gly Val Ser Thr Tyr Asn Gln Lys Phe
    290                 295                 300

Lys Asp Arg Phe Thr Ile Ser Val Asp Lys Ser Lys Asn Thr Ala Tyr
305                 310                 315                 320

Leu Gln Met Asn Ser Leu Arg Ala Glu Asp Thr Ala Val Tyr Tyr Cys
                325                 330                 335

Ala Arg Ser Gly Tyr Tyr Gly Asp Ser Asp Trp Tyr Phe Asp Val Trp
            340                 345                 350

Gly Gln Gly Thr Leu Val Thr Val Ser Ser Gly Gly Gly Gly Ser Asp
        355                 360                 365

Val Val Val Thr Gln Thr Pro Leu Ser Leu Pro Val Ser Phe Gly Asp
    370                 375                 380

Gln Val Ser Ile Ser Cys Arg Ser Ser Gln Ser Leu Ala Asn Ser Tyr
385                 390                 395                 400

Gly Asn Thr Tyr Leu Ser Trp Tyr Leu His Lys Pro Gly Gln Ser Pro
                405                 410                 415

Gln Leu Leu Ile Tyr Gly Ile Ser Asn Arg Phe Ser Gly Val Pro Asp
            420                 425                 430

Arg Phe Ser Gly Ser Gly Ser Gly Thr Asp Phe Thr Leu Lys Ile Ser

435                 440                 445

Thr Ile Lys Pro Glu Asp Leu Gly Met Tyr Tyr Cys Leu Gln Gly Thr
    450                 455                 460

His Gln Pro Tyr Thr Phe Gly Gly Gly Thr Lys Leu Glu Ile Lys Arg
465                 470                 475                 480

Ala Asp Ala Ala Ala
                485

<210>  4
<211>  24
<212>  DNA
<213>  artificial construct

<220>
<223>  CDR1

<400>  4
ggctacacat tttctgactt tgaa                                              24

<210>  5
<211>  24
<212>  DNA
<213>  artificial construct

<220>
<223>  CDR2                Heavy Chain AC133

<400>  5
attgatcctg gaactggtga tact                                              24

<210>  6
<211>  21
<212>  DNA
<213>  artificial construct

<220>
<223>  CDR3                Heavy Chain AC133

<400>  6
accctcgggg cctttgttta c                                                 21

<210>  7
<211>  18
<212>  DNA
<213>  Artificial construct

<220>
<223>  CDR1

<400>  7
caggacatcc gtaattat                                                     18

<210>  8
<211>  9
<212>  DNA
<213>  artificial construct

<220>
<223>  CDR2

<400>  8
tatacctcc                                                              9


<210>  9
<211>  27
<212>  DNA
<213>  artificial construct

<220>
<223>  CDR3

<400>  9
cagcaaggta atactctgcc gtggacg                                         27


<210>  10
<211>  24
<212>  DNA
<213>  artificial construct

<220>
<223>  CDR1

<400>  10
ggctactcct ttaccggcta cact                                            24


<210>  11
<211>  24
<212>  DNA
<213>  artificial construct

<220>
<223>  CDR2

<400>  11
attaatcctt ataaaggtgt ttcc                                            24


<210>  12
<211>  45
<212>  DNA
<213>  artificial construct

<220>
<223>  CDR3

<400>  12
gctagaagcg gatactacgg cgatagcgac tggtattttg acgtc                     45


<210>  13
<211>  33
<212>  DNA

<213>  artificial construct

<220>
<223>  CDR1

<400>  13
cagagcctgg cgaacagcta tggcaacacc tat                                    33


<210>  14
<211>  9
<212>  DNA
<213>  artificial construct

<220>
<223>  CDR2

<400>  14
ggcattagc                                                                9


<210>  15
<211>  18
<212>  DNA
<213>  artificial construct

<220>
<223>  CDR3

<400>  15
ctgcagggca cccatcag                                                     18